(19) Europäisches Patentamt
European Patent Office
Office européen des brevets

(11) Veröffentlichungsnummer: **0 241 735 A2**

(12) **EUROPÄISCHE PATENTANMELDUNG**

(21) Anmeldenummer: **87103869.1**

(22) Anmeldetag: **17.03.87**

(51) Int. Cl.⁴: **C 07 D 333/38**, A 01 N 43/10

(30) Priorität: **29.03.86 DE 3610711**

(43) Veröffentlichungstag der Anmeldung: **21.10.87 Patentblatt 87/43**

(84) Benannte Vertragsstaaten: **AT BE CH DE FR GB IT LI NL**

(71) Anmelder: **BAYER AG, Konzernverwaltung RP Patentabteilung, D-5090 Leverkusen 1 Bayerwerk (DE)**

(72) Erfinder: **Daum, Werner, Dr., Bärenstrasse 18, D-4150 Krefeld (DE)**
Erfinder: **Hänssler, Gerd, Dr., Am Arenzberg 58a, D-5090 Leverkusen 3 (DE)**

(54) **Acyloxythiophen-carbonamide.**

(57) Neue Acyloxythiophen-carbonamide der Formel (I)

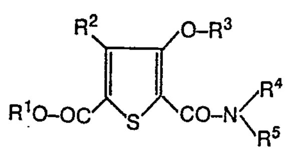

(I)

in welcher

$R^1$, $R^2$, $R^3$, $R^4$ und $R^5$ die in der Beschreibung gegebenen Bedeutungen haben und ihre Verwendung zur Bekämpfung von Schädlingen.

Die neuen Acyloxythiophen-carbonamide der Formel (I) können z.B. hergestellt werden, wenn man geeignete 2-alkoxycarbonylsubstituierte 4-Hydroxythiophen-5-carbonamide mit geeigneten Acylierungsmitteln oder mit geeigneten Isocyanaten umsetzt. Einige können auch hergestellt werden, wenn man geeignete Hydroxythiophen-carbonamide in 1. Stufe mit Phosgen und in einer 2. Stufe mit geeigneten Aminen umsetzt.

EP 0 241 735 A2

BAYER AKTIENGESELLSCHAFT
Konzernverwaltung RP
Patentabteilung

5090 Leverkusen, Bayerwerk

Bas/bo/c
Ib

## Acyloxythiophen-carbonamide

Die vorliegende Erfindung betrifft neue Acyloxythiophen-carbonamide, Verfahren zu ihrer Herstellung und ihre Verwendung als Schädlingsbekämpfungsmittel, vor allem als Fungizide.

Es ist bereits bekannt, daß 2,5-Bis-(alkoxycarbonyl)-3,4-bis-(acyloxy)-thiophene und 2,5-Bis-(alkoxycarbonyl)-3-alkyl-4-acyloxythiophene fungizide Eigenschaften besitzen (vgl. EP 32 748 und EP 93 384). Hier ist besonders das 2,5-Bis-(isopropoxycarbonyl)-3-methyl-4-(3-methylbenzoyloxy)-thiophen zu nennen, das bekannt ist aus T. Wada et al., Proceedings of the 10th Intern. Congress of Plant Protection, 20.-25.11.1983, Brighton, Vol 1, 400-407.

Es wurden neue Acyloxythiophen-carbonamide der allgemeinen Formel (I)

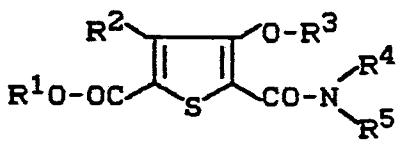

(I)

in welcher

$R^1$ für Alkyl, Alkoxyalkyl, Alkylthioalkyl, Fluoralkyl, Cyanalkyl, Alkenyl, Alkinyl oder für Cycloalkyl steht,

$R^2$ für Alkyl oder gegebenenfalls substituiertes Phenyl steht,

$R^3$ für Alkylsulfonyl, Halogenalkylsulfonyl, für gegebenenfalls substituiertes Phenylsulfonyl oder für den Rest -OC-Q steht, in dem

Q für Alkyl steht, das gegebenenfalls gleich oder verschieden durch Halogen, Alkoxy, Alkylthio, Alkylsulfonyl, Alkylthioalkoxy, Alkoxycarbonyl, Phenyl, Phenoxy und Halogenphenoxy substituiert ist; für Alkenyl, das gegebenenfalls gleich oder verschieden durch Halogen, Phenyl, Halogenphenyl und Halogenalkylphenyl substituiert ist; für Alkinyl; für Cycloalkyl; für Alkoxy, das gegebenenfalls substituiert ist durch Halogen oder Cyan; für Phenyl, das gegebenenfalls gleich oder verschieden substituiert ist durch Halogen, Alkyl, Halogenalkyl, Alkoxy, Halogenalkoxy, Alkylthio, Halogenalkylthio, Alkylsulfinyl, Halogenalkylsulfinyl, Alkylsulfonyl und Halogenalkylsulfonyl; für Pyridyl; für Monoalkylamino, wobei der Alkylteil gegebenenfalls substituiert ist durch Halogen, Cyan, Alkoxycarbonyl, Dialkylaminocarbonyl, Alkanylenaminocarbonyl oder Oxaalkanylenaminocarbonyl; für 1-Cyancycloalkylamino; für Dialkylamino; für Alkanylenamino; für Oxaalkanylenamino; für N-Alkyl-N-phenylamino oder Phenylamino steht, worin jeweils der Phenylrest gegebenenfalls gleich oder verschieden substituiert ist durch Halogen, Alkyl, Halogenalkyl, Alkoxy, Halogenalkoxy, Alkylthio, Halogenalkylthio, Alkylsulfonyl, Halogenalkylsulfonyl, Alkanylendioxy, Halogenalkanylendioxy oder durch $-O-CHal_2-O-CHal_2-$ oder für Alkoxycarbonylamino steht,

$R^4$ für Alkyl, Alkoxyalkyl, Alkylthioalkyl, Cyanalkyl, Fluoralkyl, Alkenyl, Alkinyl, Cycloalkyl oder Alkoxy steht,

$R^5$ für Wasserstoff, Alkyl, Alkoxyalkyl, Alkylthioalkyl, Fluoralkyl, Cyanalkyl, Alkenyl, Alkinyl oder Cycloalkyl steht,

oder

$R^4$ und $R^5$ gemeinsam mit dem Stickstoffatom, an dem sie stehen, für einen gegebenenfalls substituierten Heterocyclus stehen, welcher in der Alkylenkette durch weitere Heteroatome substituiert sein kann,

gefunden.

Weiterhin wurde gefunden, daß man die neuen Acyloxythiophen-carbonamide der Formel (I)

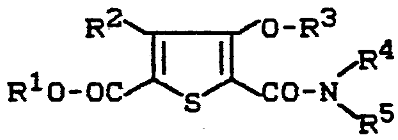

(I)

in welcher

$R^1$ für Alkyl, Alkoxyalkyl, Alkylthioalkyl, Fluoralkyl, Cyanalkyl, Alkenyl, Alkinyl oder für Cycloalkyl steht,

$R^2$ für Alkyl oder gegebenenfalls substituiertes Phenyl steht,

$R^3$ für Alkylsulfonyl, Halogenalkylsulfonyl, für gegebenenfalls substituiertes Phenylsulfonyl oder für den Rest -OC-Q steht, in dem

Q für Alkyl steht, das gegebenenfalls gleich oder verschieden durch Halogen, Alkoxy, Alkylthio, Alkylsulfonyl, Alkylthioalkoxy, Alkoxycarbonyl, Phenyl, Phenoxy und Halogenphenoxy substituiert ist; für Alkenyl, das gegebenenfalls gleich oder verschieden durch Halogen, Phenyl, Halogenphenyl und Halogenalkylphenyl substituiert ist; für Alkinyl; für Cycloalkyl; für Alkoxy, das gegebenenfalls substituiert ist durch Halogen oder Cyan; für Phenyl, das gegebenenfalls

gleich oder verschieden substituiert ist durch Halogen, Alkyl, Halogenalkyl, Alkoxy, Halogenalkoxy, Alkylthio, Halogenalkylthio, Alkylsulfinyl, Halogenalkylsulfinyl, Alkylsulfonyl und Halogenalkylsulfonyl; für Pyridyl; für Monoalkylamino, wobei der Alkylteil gegebenenfalls substituiert ist durch Halogen, Cyan, Alkoxycarbonyl, Dialkylaminocarbonyl, Alkanylenaminocarbonyl oder Oxaalkanylenaminocarbonyl; für 1-Cyancycloalkylamino; für Dialkylamino; für Alkanylenamino; für Oxaalkanylenamino; für N-Alkyl-N-phenylamino oder Phenylamino steht, worin jeweils der Phenylrest gegebenenfalls gleich oder verschieden substituiert ist durch Halogen, Alkyl, Halogenalkyl, Alkoxy, Halogenalkoxy, Alkylthio, Halogenalkylthio, Alkylsulfonyl, Halogenalkylsulfonyl, Alkanylendioxy, Halogenalkanylendioxy oder durch $-O-CHal_2-O-CHal_2-$ oder für Alkoxycarbonylamino steht,

$R^4$ für Alkyl, Alkoxyalkyl, Alkylthioalkyl, Cyanalkyl, Fluoralkyl, Alkenyl, Alkinyl, Cycloalkyl oder Alkoxy steht,

$R^5$ für Wasserstoff, Alkyl, Alkoxyalkyl, Alkylthioalkyl, Fluoralkyl, Cyanalkyl, Alkenyl, Alkinyl oder Cycloalkyl steht,

oder

$R^4$ und $R^5$ gemeinsam mit dem Stickstoffatom, an dem sie stehen, für einen gegebenenfalls substituierten Heterocyclus stehen, welcher in der Alkylenkette durch weitere Heteroatome substituiert sein kann,

erhält, wenn man

a) ein 4-Hydroxythiophen-5-carbonamid der Formel (II)

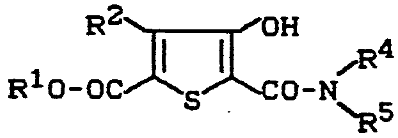

(II)

in welcher

$R^1$, $R^2$, $R^4$ und $R^5$ die obengenannten Bedeutungen haben,

mit einem Acylierungsmittel der Formel (III)

$$X-R^3 \quad \text{(III)}$$

in der

$R^3$ die vorstehend genannte Bedeutung hat und

X für eine Abgangsgruppe, wie z.B. für Halogen steht,

umsetzt,

oder

b) Verbindungen der Formel (II) mit einem Isocyanat der Formel (IV)

OCN-Y (IV)

in welcher

Y für Alkyl steht, das gegebenenfalls gleich oder verschieden durch Halogen, Cyan, Alkoxycarbonyl, Dialkylaminocarbonyl, Alkanylenaminocarbonyl, Oxaalkanylenaminocarbonyl substituiert ist; für 1-Cyancycloalkyl, Alkoxycarbonyl oder für Phenyl steht, das gegebenenfalls gleich oder verschieden substituiert ist durch Halogen, Alkyl, Halogenalkyl, Alkoxy, Halogenalkoxy, Alkylthio, Halogenalkylthio, Alkylsulfonyl, Halogenalkylsulfonyl, Alkanylendioxy, Halogenalkanylendioxy oder durch den Rest $-O-CHal_2-O-CHal_2-$,

umsetzt,

oder wenn man

c) für den Fall, daß $R^3$ ein substituierter Aminocarbonylrest ist, in einer 1. Stufe ein Hydroxy-thiophencarbonamid der Formel (II) mit Phosgen in Gegenwart einer tertiären organischen Base umsetzt und das gebildete 4-(Chlorcarbonyloxy)-thiophen-5-carbonamid-Derivat der Formel (V)

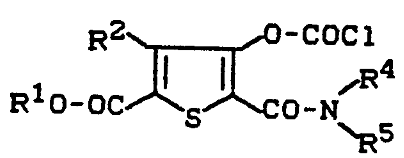

(V)

in der

$R^1$, $R^2$, $R^4$ und $R^5$ die obengenannten Bedeutungen haben,

mit einem Amin der Formel (VI)

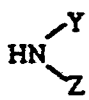 (VI)

in der

Y die oben angegebene Bedeutung hat und

Z für Wasserstoff oder für Alkyl steht oder

Y und Z zusammen für Alkanylen oder Oxyalkanylen stehen,

gegebenenfalls in Gegenwart eines Lösungs- oder Verdünnungsmittels und gegebenenfalls in Gegenwart einer tertiären organischen Base, umsetzt.

Weiterhin wurde gefunden, daß die neuen Acyloxythiophencarbonamide der Formel (I) biologisch aktiv sind.

Überraschenderweise zeigen die erfindungsgemäßen Acyloxythiophen-carbonamide der Formel (I) eine bessere Wirksamkeit gegen Schädlinge als die aus dem Stand der Technik bekannten Verbindungen gleicher Wirkungsrichtung oder konstitutionell ähnlichen Verbindungen.

Die erfindungsgemäßen Acyloxythiophen-carbonamide sind durch die Formel (I) allgemein definiert. Bevorzugt sind Verbindungen der Formel (I), bei welchen

$R^1$ für Alkyl mit 1 bis 5 Kohlenstoffatomen, für Alkoxyalkyl oder für Alkylthioalkyl mit je 1 bis 5 Kohlenstoffatomen je Alkylteil, für Fluoralkyl mit 1 bis 5 Kohlenstoffatomen und 1 bis 5 Fluoratomen, für Cyanalkyl mit 1 bis 5 Kohlenstoffatomen im Alkylteil, für Alkenyl mit 3 oder 4 Kohlenstoffatomen, für Alkinyl mit 3 bis 5 Kohlenstoffatomen oder für Cycloalkyl mit 4 bis 6 Kohlenstoffatomen steht,

$R^2$ für Alkyl mit 1 bis 4 Kohlenstoffatomen oder für gegebenenfalls ein- bis fünffach, gleich oder verschieden substituiertes Phenyl steht, wobei als Substituenten Alkyl mit 1 bis 4 Kohlenstoffatomen, Alkoxy oder Alkylthio mit je 1 bis 4 Kohlenstoffatomen, Halogen, Nitro, Halogenalkyl, Halogenalkoxy oder Halogenalkylthio mit je 1 bis 4 Kohlenstoffatomen und 1 bis 5 gleichen oder verschiedenen Halogenatomen in Frage kommen. Halogen bedeutet Fluor, Chlor, Brom und Iod, vorzugsweise Fluor und Chlor, in allen vorhergehenden und folgenden Definitionen, wenn nicht anders definiert,

$R^3$ für Alkylsulfonyl mit 1 bis 4 Kohlenstoffatomen, für Halogenalkylsulfonyl mit 1 bis 4 Kohlenstoffatomen und 1 bis 4 gleichen oder verschiedenen Halogenatomen, für Phenylsulfonyl steht, wobei der Phenylrest 1- bis 5-fach, gleich oder verschieden durch Halogen, Alkyl mit 1 bis 4 Kohlenstoffatomen oder Nitro substituiert sein kann oder für den Rest -OC-Q steht, in dem

Q für Alkyl mit 1 bis 4 Kohlenstoffatomen steht und gegebenenfalls durch bis zu 4 gleichen oder verschiedenen Halogenatomen, Alkoxy, Alkylthio, Alkylsulfonyl, Alkoxycarbonyl mit je bis zu 4 Kohlenstoffatomen, Alkylthioalkoxy mit je bis zu 2 Kohlenstoffatomen je Alkyleinheit, Phenyl, Phenoxy oder Halogenphenoxy mit bis zu 3 gleichen oder verschiedenen Halogenatomen substituiert sein kann; für Alkenyl mit 2 bis 4 Kohlenstoffatomen steht und gegebenenfalls substituiert sein kann durch 1 bis 4 gleiche oder verschiedene Halogenatome, Phenyl, Halogenphenyl mit 1 bis 3 gleichen oder verschiedenen Halogenatomen oder Halogenalkylphenyl mit 1 bis 3 gleichen oder verschiedenen Halogenatomen und 1 oder 2 Kohlenstoffatomen im Halogenalkylrest; für Alkinyl mit bis zu 3 Kohlenstoffatomen; für Cycloalkyl mit 3 bis 6 Kohlenstoffatomen; für Alkoxy mit 1 bis 4 Kohlenstoffatomen, das gegebenenfalls substituiert sein kann mit bis zu 3 gleichen oder verschiedenen Halogenatomen oder Cyan; für Phenyl, das gegebenenfalls gleich oder verschieden, ein- bis dreifach substituiert sein kann

durch gleiche oder verschiedene Halogenatome, Alkyl mit 1 bis 4 Kohlenstoffatomen, Halogenalkyl mit 1 oder 2 Kohlenstoffatomen und 1 bis 3 gleichen oder verschiedenen Halogenatomen, Alkoxy mit 1 oder 2 Kohlenstoffatomen, Halogenalkoxy mit 1 oder 2 Kohlenstoffatomen und 1 bis 3 gleichen oder verschiedenen Halogenatomen, Alkylthio mit 1 oder 2 Kohlenstoffatomen, Halogenalkylthio mit 1 oder 2 Kohlenstoffatomen und 1 bis 3 gleichen oder verschiedenen Halogenatomen, Alkylsulfinyl mit 1 oder 2 Kohlenstoffatomen, Halogenalkylsulfinyl mit 1 oder 2 Kohlenstoffatomen und 1 bis 3 gleichen oder verschiedenen Halogenatomen, Alkylsulfonyl mit 1 oder 2 Kohlenstoffatomen oder Halogenalkylsulfonyl mit 1 oder 2 Kohlenstoffatomen und 1 bis 3 gleichen oder verschiedenen Halogenatomen; für Pyridyl; für Alkylamino mit 1 bis 6 Kohlenstoffatomen, das gegebenenfalls in der Alkylgruppe mit 1 bis 4 gleichen oder verschiedenen Halogenatomen, Cyan, Alkoxycarbonyl mit 1 bis 6 Kohlenstoffatomen in der Alkoxygruppe, Dialkylaminocarbonyl mit je 1 bis 3 Kohlenstoffatomen je Alkylteil, Alkanylenaminocarbonyl oder Oxaalkanylenaminocarbonyl mit je bis zu 6 Kohlenstoffatomen im Alkanylenteil substituiert sein kann; für 1-Cyancycloalkylamino mit 5 oder 6 Kohlenstoffatomen im Cycloalkylteil; für Dialkylamino mit 1 bis 3 Kohlenstoffatomen je Alkylteil, Alkanylenamino und Oxaalkanylenamino mit je bis zu 6 Kohlenstoffatomen, N-Alkyl-N-phenylamino mit 1 bis 3 Kohlenstoffatomen im Alkylteil oder für Phenylamino, wobei jeweils der Phenylrest gleich oder verschieden, ein- bis dreifach substituiert sein kann durch gleiche oder verschiedene Halogenatome, durch Alkyl, Alkoxy, Alkylthio und Alkylsulfonyl mit je 1 oder 2 Kohlenstoffatomen, Halogenalkyl, Halogenalkoxy, Halogenalkylthio und Halogenalkylsulfonyl mit je 1 oder 2 Kohlenstoffatomen und 1 bis 3 gleichen oder verschiedenen Halogenatomen, Alkanylendioxy mit 1 oder 2 Kohlenstoffatomen, Halogenalkanylendioxy mit 1 oder 2 Kohlenstoffatomen und bis zu 4 Halogenatomen oder durch den Rest $-O-CHal_2-O-CHal_2-$ oder für Alkoxycarbonylamino mit 1 bis 8 Kohlenstoffatomen im Alkylteil steht,

$R^4$ für Alkyl mit 1 bis 5 Kohlenstoffatomen, für Alkoxyalkyl oder Alkylthioalkyl mit jeweils 1 bis 5 Kohlenstoffatomen je Alkylteil, für Cyanalkyl mit 1 bis 5 Kohlenstoffatomen im Alkylteil, für Fluoralkyl mit 1 bis 3 Fluoratomen und 1 bis 5 Kohlenstoffatomen, für Alkenyl oder Alkinyl mit je 3 bis 5 Kohlenstoffatomen, für Cycloalkyl mit 3 bis 6 Kohlenstoffatomen oder für Alkoxy mit 1 bis 5 Kohlenstoffatomen steht,

$R^5$ für Wasserstoff, Alkyl mit 1 bis 5 Kohlenstoffatomen, für Alkoxyalkyl oder Alkylthioalkyl mit jeweils 1 bis 5 Kohlenstoffatomen je Alkylteil, für Cyanalkyl mit 1 bis 5 Kohlenstoffatomen im Alkylteil, für Fluoralkyl mit 1 bis 3 Fluoratomen und 1 bis 5 Kohlenstoffatomen, für Alkenyl oder Alkinyl mit 3 bis 5 Kohlenstoffatomen, für Cycloalkyl mit 6 Kohlenstoffatomen steht, oder

$R^4$ und $R^5$ zusammen mit dem Stickstoffatom für einen 5-, 6- oder 7-gliedrigen heterocyclischen Ring stehen, der Aza-, Oxa- oder Thiaelemente enthalten und durch Alkylgruppen mit 1 bis 4 Kohlenstoffatomen gleich oder verschieden substituiert sein kann.

Besonders bevorzugt sind Verbindungen der Formel (I), bei welchen

$R^1$ für Methyl, Ethyl, n- oder iso-Propyl, 2,2-Dimethylpropyl, 2-Methoxyethyl, 2-Ethoxyethyl, 2-Methylthioethyl, 2-Ethylthioethyl, 2,2,2-Trifluorethyl, Cyanmethyl, Cyanethyl, Allyl, Methallyl, 2-Propinyl, 1,1-Dimethyl-2-propinyl, Cyclopentyl oder Cyclohexyl steht,

$R^2$ für Methyl, Ethyl, n-Propyl, iso-Propyl, n-Butyl, sek.-Butyl, iso-Butyl oder tert.-Butyl, Phenyl oder 1- bis 3-fach, gleich oder verschieden durch Halogen substituiertes Phenyl steht,

$R^3$ für Methansulfonyl, Chlormethansulfonyl, Phenylsulfonyl, 2-, 3- und 4-Chlorphenylsulfonyl, 3,4-Dichlorphenylsulfonyl, 2- und 4-Methylphenylsulfonyl, 3-Chlor-4-methylphenylsulfonyl, 2-, 3- und 4-Nitrophenylsulfonyl, 2-Chlor-5-nitrophenylsulfonyl, 2,4,5-Trifluor-3-nitrophenylsulfonyl, 2-Methyl-5-nitrophenylsulfonyl oder für den Rest -OC-Q steht, wobei

Q für Methyl, Ethyl, Isopropyl, Butyl, Dichlormethyl, Trichlormethyl, Trifluormethyl, 1-Brom-1-methylethyl, Methoxymethyl, Butoxymethyl, 2-Butylthioethyl, Propylsulfonylmethyl, Methoxycarbonylethyl, Butoxycarbonylethyl, Methylthioethoxymethyl, Benzyl, Phenoxymethyl, 1-Phenoxy-ethyl, 1-(4-Chlorphenoxy)-ethyl, 2,4-Dichlor-phenoxymethyl; Vinyl, Propen-1-yl, 2-Chlorvinyl, 2-(Phenyl)-vinyl, 2-(4-Chlorphenyl)-vinyl, 2-(4-Chlorphenyl)-2-chlorvinyl, 2-(2-Trifluormethylphenyl)-vinyl, Ethinyl, 2-Methyl-ethinyl, Cyclopropyl, Cyclobutyl, Cyclopentyl, Cyclohexyl, Methoxy, Isopropoxy, Butoxy, 2-Chlorethoxy, 2,2,2-Trichlorethoxy, 2,2,2-Trifluorethoxy, 2-Cyanethoxy; Phenyl, 2-, 3- oder 4-Chlorphenyl, 2-Bromphenyl, 4-Fluorphenyl, 2-, 3- oder 4-Methylphenyl, 4-tert.-Butylphenyl, 2-, 3- oder 4-Trifluormethylphenyl, 3-Chlor-4-trifluormethylphenyl, 4-Methoxyphenyl, 3-Trifluormethoxyphenyl, 3-Chlor-4-trifluormethoxyphenyl, 4-Chlordifluormethoxyphenyl, 4-Ethylthiophenyl, 4-Trifluormethylthiophenyl, 4-Ethylsulfinylphenyl, 4-Trifluormethylsulfinylphenyl, 4-Methylsulfonylphenyl, 4-Trifluormethylsulfonylphenyl; 2-, 3- oder 4-Pyridyl; Methylamino, Butylamino, Hexylamino, 2,2,2-Trifluorethylamino, 1-Trifluormethyl-ethylamino, 6-Chlorhexylamino, 2-Cyanethylamino, 1-Cyan-1-methylethylamino, 1-Cyan-1-methylpropylamino, 1-Cyano-1-ethylpropylamino, 1-Cyan-cyclopent-1-ylamino, 1-Cyancyclohex-1-ylamino, 3-Cyanpropylamino, 5-Cyanpentylamino, 6-Cyanhexylamino, Methoxycarbonylmethylamino, Ethoxycarbonylmethylamino, Butylcarbonylethylamino,

Isobutoxycarbonylethylamino, 1-Methoxycarbonyl-1-methyl-ethylamino, 1-Propoxycarbonyl-1-methyl-ethylamino, 1-Ethoxycarbonyl-1-ethyl-ethylamino, 1-Isobutoxycarbonyl-1-ethyl-ethylamino, Methoxycarbonylpropylamino, Methoxycarbonyl-pentylamino, Isopropoxycarbonyl-pentylamino, sek.-Butyloxycarbonyl-pentylamino, 2-Ethoxycarbonyl-2-ethyl-butylamino, Butoxycarbonylpentylamino, 5-(2,2-Dimethylpropyloxycarbonyl)-pentylamino, N-Morpholinocarbonylmethylamino, 1-(N,N-Diethylaminocarbonyl)-ethylamino, 2-(N-Pyrrolidinocarbonyl)-ethylamino, 3-(N-Piperidinocarbonyl)-propylamino, 5-(N,N-Dimethylaminocarbonyl)-pentylamino; 1-Cyancyclohex-1-ylamino, Dimethylamino, Diethylamino, Pyrrolidino, Piperidino, 3,5-Dimethylmorpholino, N-Methyl-N-phenylamino, Phenylamino, 2-Chlorphenylamino, 2,4,5-Trichlorphenylamino, 4-Fluorphenylamino, 2-, 3- oder 4-Methylphenylamino, 3,5-Dimethylphenylamino, 2-, 3- oder 4-Trifluormethylphenylamino, 2-Chlor-5-trifluormethylphenylamino, 4-Ethoxyphenylamino, 3,5-Dichlor-4-methoxyphenylamino, 4-Trifluormethoxyphenylamino, 4-Ethylthiophenylamino, 3-Trifluormethylthiophenylamino, 4-Ethylsulfonylphenylamino, 3-Trifluormethylsulfonylphenylamino, 3,4-Methylendioxyphenylamino, 3,4-Difluormethylendioxyphenylamino, 3,4-(Tetrafluorethylendioxy)-phenylamino, 2,2,4,4-Tetrafluorbenzodiox-1,3-en-6-ylamino; Methoxycarbonylamino, Ethoxycarbonylamino, Butoxycarbonylamino und 2-Ethylhexoxycarbonylamino steht,

$R^4$ für Methyl, Ethyl, n- oder iso-Propyl, 2,2-Dimethylpropyl, 2-Methoxyethyl, 2-Ethoxyethyl, 2-Propoxyethyl, 3-Methoxypropyl, 3-Ethoxypropyl, 3-Butoxypropyl, 2-Methylthioethyl, 2-Ethylthioethyl, 2,2,2-Trifluorethyl, 2-Cyanethyl, 1-Methyl-1-cyanoethyl, ω-Cyanpentyl, Allyl, Methallyl, 2-Propinyl, 1,1-Dimethyl-2-propinyl, Cyclopropyl, Cyclobutyl, Cyclopentyl, Cyclohexyl, Methoxy, Ethoxy, n-Propyloxy, n-Butyloxy, sek.-Butyloxy oder n-Pentyloxy steht,

$R^5$ für Wasserstoff, Methyl, Ethyl, n- oder iso-Propyl, 2,2-Dimethylpropyl, 2-Methoxyethyl, 2-Ethoxyethyl, 2-Propoxyethyl, 3-Methoxypropyl, 3-Ethoxypropyl, 3-Butoxypropyl, 2-Methylthioethyl, 2-Ethylthioethyl, 2,2,2-Trifluorethyl, 2-Cyanethyl, 1-Methyl-1-cyanoethyl, ω-Cyanpentyl, Allyl, Methallyl, 2-Propinyl, 1,1-Dimethyl-2-propinyl, Cyclohexyl steht, oder

$R^4$ und $R^5$ zusammen mit dem Stickstoffatom für Pyrrolidin, Piperidin, 2-Methylpiperidin, 3-Methylpiperidin, 4-Methylpiperidin, Hexahydro-1H-azepin, Morpholin, 2,6-Dimethylmorpholin, Thiazolidin, $N^1$-Methylpiperazin oder $N^1$-Propylpiperazin stehen.

Insbesondere seien Verbindungen der Formel (I) genannt, in denen

$R^1$ für Methyl, Ethyl, Isopropyl, n-Propyl, sek.-Butyl, Cyclopentyl oder Cyclohexyl steht,

$R^2$ für Methyl, Ethyl, Isopropyl, tert.-Butyl oder Phenyl steht,

$R^3$ für Methansulfonyl, Chlormethansulfonyl, Chlorethansulfonyl, p-Toluylsulfonyl, Benzoyl, 3-Methyl-, 4-Fluor-, 3-Trifluormethylbenzoyl; Methylaminocarbonyl, N,N-Dimethyl-, 2-Methyl-2-cyanethyl-, 5-Cyanpentyl-, 5-Butoxycarbonylpentyl-, 5-(2,2-Dimethylpropoxycarbonyl)-pentyl-aminocarbonyl; 2-, 3- und 4-Toluylaminocarbonyl; 3-Trifluormethyl-, 3-Trifluormethoxyphenylaminocarbonyl oder 2-Ethylhexoxycarbonyl-aminocarbonyl steht,

$R^4$ für Methyl, Methoxyethyl, Methoxypropyl oder Cyanpentyl steht und

$R^5$ für Wasserstoff steht oder

$R^4$ und $R^5$ zusammen für Butanylen, Pentanylen oder 3-Oxapentanylen stehen.

Verwendet man als Ausgangsstoffe zur Herstellung der erfindungsgemäßen Verbindungen der Formel (I) nach Verfahrensvariante a) beispielsweise 2-Methoxycarbonyl-3-methyl-4-hydroxy-5-dimethylaminocarbonylthiophen und p-Fluorbenzoylchlorid sowie Pyridin als Lösungsmittel und als Chlorwasserstoffakzeptor, bzw. Methylisocyanat nach Verfahrensvariante b), so können die Reaktionsverläufe durch die folgenden Formelschemata wiedergegeben werden:

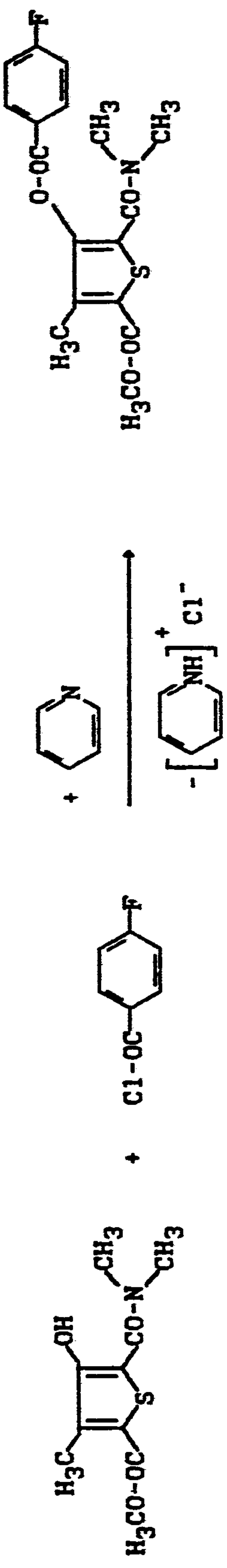

oder

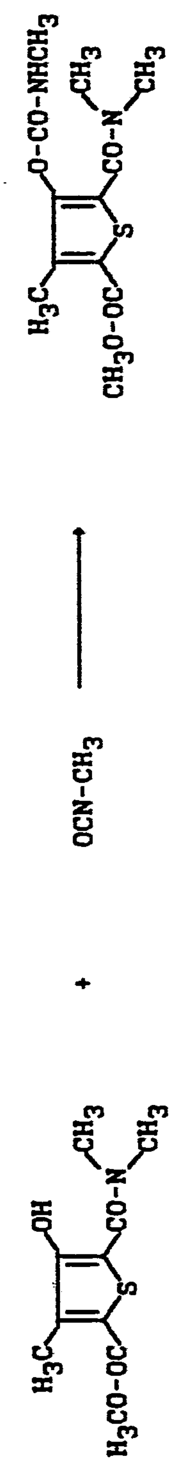

Die als Ausgangsstoffe zur Durchführung des erfindungsgemäßen Verfahrens a) benötigten Sulfonsäure- und Carbonsäure-Derivate sind durch die Formel (III) allgemein definiert. Die Verbindungen sind überwiegend bekannt und können nach bekannten Verfahren hergestellt werden. Zu nennen sind hier insbesondere: Methan-, Chlormethan-, 4-Chlorbutan-, Phenylsulfonylchlorid, 2-, 3- und 4-Chlorphenylsulfonylchlorid, 3,4-Dichlor-, 2-Methyl-5-chlor-, 2- und 4-Methylphenylsulfonylchlorid, 2-, 3- und 4-Nitrophenylsulfonsäurechlorid, 2-Chlor-5-nitrophenylsulfonsäurechlorid, 2,4,5-Trifluor-3-nitrophenylsulfonsäurechlorid, 2-Methyl-5-nitrophenylsulfonsäurechlorid; Acetylbromid, Isobuttersäurebromid, 2,2-Dimethylpropanoylchlorid, Trifluoracetylchlorid, 4-Brombutyroylchlorid, 2,3-Dibrom-propionylchlorid, Methoxy- bzw. Butoxyacetylchlorid, Ethylthioacetylchlorid, Isopropylsulfonylacetylchlorid, 3-(Methoxycarbonyl)-propionylchlorid, Methylthioethyloxyacetylchlorid, 2-Phenylpropionylchlorid, 4-Chlorphenylacetylchlorid, 2,4,5-Trichlorphenoxyacetylchlorid, Methacryloylchlorid, Crotonoylchlorid, 2,3-Dibromacryloylchlorid, 3,4,4- bzw. 4,4,4-Trichlorcrotonoylchlorid, Zimtsäurechlorid, 4-Chlorzimtsäurechlorid, 3-(2-Trifluormethylphenyl)-acryloylchlorid, 3-(4-Chlorphenyl)-3-chloracryloylchlorid, Propargylsäurechlorid, Cyclopropyl-, Cyclopentyl- bzw. Cyclohexylcarbonsäurechlorid; Methoxy-, Isopropoxy-, 2-Chlorethoxy-, 2,2,2-Trifluorethoxy-, 2,2,2-Trichlorethoxy-, 2,3-Tribrompropoxy- bzw. 2-Cyanethoxycarbonylchlorid; Benzoylchlorid, 2-, 3- bzw. 4-Chlor-, 3,4-Dichlor-, 4-Fluor- und 3,5-Dichlor-4-fluorbenzoylchlorid;

2-, 3- bzw. 4-Toluylsäurechlorid, 4-tert.-Butyl-, 2-, 3- bzw. 4-Trifluormethyl-, 2-Methoxy-, 3,5-Dimethoxy-, 2-Methoxy-6-trifluormethyl-, 4-Ethylthio-, 4-Ethylsulfinyl- bzw. 4-Ethylsulfonylbenzoylchlorid; 3- und 4-Trifluormethoxy-, 3-Trifluormethyl-4-isopropyl-, 3-Chlor-4-trifluormethoxy-, 4-Chlordifluormethoxy-, 4-Chlordifluormethylthio-, 4-Trifluormethylsulfinyl- bzw. 4-Trifluormethylsulfonylbenzoylhalogenid; 2-, 3- bzw. 4-Pyridincarbonsäurechlorid-hydrochlorid; N,N-Dimethylamino-, N,N-Dipropylamino-, N-Methyl-N-phenylamino- bzw. N-Ethyl-N-phenylamino-carbonylchlorid; N-Chlorcarbonyl-pyrrolidin, -piperidin, -3-methylpiperidin, -morpholin bzw. -3,5-dimethylmorpholin.

Die als Ausgangsstoffe zur Durchführung des erfindungsgemäßen Verfahrens benötigten Isocyanate sind durch die Formel (IV) allgemein definiert. Die Verbindungen sind überwiegend bekannt und können nach bekannten Verfahren hergestellt werden. Die Isocyanate können z.B. hergestellt werden durch Umsetzen der Aminoverbindungen mit Phosgen (Liebigs Ann. Chem. <u>562</u>, 75-136, (1949)), durch Umsetzen von Chlorcarbonylisocyanat mit Alkoholen bzw. mit Aminen (DOS 1 913 273) sowie durch thermische Spaltung der Phenoxycarbonylamino-Verbindungen.

Zu nennen sind hier insbesondere:
Methylisocyanat, Butylisocyanat, Hexylisocyanat, 2,2,2-Trifluorethylisocyanat, 1-(Trifluormethyl)-ethylisocyanat, 6-Chlorhexylisocyanat, 2-Cyanethyl-isocyanat, 1-Cyan-1-methylethyl-isocyanat, 1-Cyan-1-methyl-propylisocyanat, 1-Cyan-1-ethyl-propylisocyanat, 1-Cyan-cyclopent-1-yl-isocyanat, 1-Cyan-cyclohex-1-ylisocyanat, 3-Cyanpropyl-isocyanat, 5-Cyan-pentylisocyanat, 6-Cyanhexyl-isocyanat, N-Morpholino-carbonylmethylisocyanat, 1-(N,N-Diethylaminocarbonyl)-ethylisocyanat, N-Pyrrolidino-carbonylethylisocyanat, N-Piperidino-carbonylpropylisocyanat, N,N-Dimethylaminocarbonyl-pentylisocyanat, Methoxycarbonylmethyl-isocyanat, Ethoxycarbonylmethyl-isocyanat, Butoxycarbonylethyl-isocyanat, Isobutoxycarbonylethyl-isocyanat, 1-Methoxycarbonyl-1-methyl-ethyl-isocyanat, 1-Propoxycarbonyl-1-methyl-ethyl-isocyanat, 1-Ethoxycarbonyl-1-ethyl-ethyl-isocyanat, 1-Isobutoxycarbonyl-1-ethyl-ethyl-isocyanat, Methoxycarbonyl-propyl-isocyanat, Methoxycarbonyl-pentyl-isocyanat, Isopropoxycarbonyl-pentyl-isocyanat, sek.-Butyloxycarbonyl-pentyl-isocyanat, Allyloxycarbonyl-pentyl-isocyanat, Propargyloxycarbonylpentyl-isocyanat, 2-Ethoxycarbonyl-2-ethyl-butyl-isocyanat, Butoxycarbonylpentyl-isocyanat, 2,2-Dimethylpropyloxycarbonyl-pentylisocyanat, Methoxycarbonylisocyanat, Ethoxycarbonylisocyanat, 2,2-Dimethylpropyloxycarbonylisocyanat, 2-Ethylhexyloxycarbonyl-isocyanat, Octyloxycarbonyl-isocyanat, Phenylisocyanat, 2-Chlorphenylisocyanat, 2,4,5-Trichlorphenylisocyanat, 4-Fluorphenylisocyanat, 2-, 3- bzw. 4-Methylphenylisocyanat, 3,5-Dimethylphenylisocyanat, 2-, 3- bzw. 4-Trifluormethylphenylisocyanat, 2-Chlor-5-trifluormethylphenylisocyanat, 4-Ethoxyphenylisocyanat, 3,5-Dichlor-4-methoxyphenylisocyanat, 4-Trifluormethoxyphenylisocyanat, 4-Ethoxythiophenylisocyanat, 3-Trifluormethylthiophenylisocyanat, 4-Ethylsulfonylphe-

nylisocyanat, 3-Trifluormethylsulfonylphenylisocyanat, 3,4-Methylendioxyphenylisocyanat, 3,4-Difluormethylendioxyphenylisocyanat, 3,4-(Tetrafluorethylendioxy)-phenylisocyanat, 2,2,4,4-Tetrafluorbenzodiox-1,3-en-6-yl-isocyanat.

Weiterhin werden für die Umsetzungen zu den erfindungsgemäßen Verbindungen 4-Hydroxythiophen-5-carbonamide benötigt, die durch die Formel (II) allgemein definiert sind. Diese sind noch nicht bekannt; sie sind Gegenstand eigener, nicht vorveröffentlichter Anmeldungen (vgl. P 35 23 313.3 vom 29.06.1985 und P 36 02 889.4 vom 31.01.1986). Sie werden erhalten, indem man 3-substituierte 2,5-Bis-(alkoxycarbonyl)-4-hydroxythiophene der allgemeinen Formel (VII)

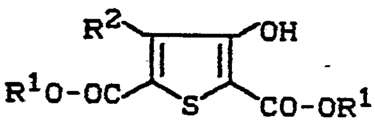

(VII)

in der

$R^1$ und $R^2$ die vorstehend genannte Bedeutung haben,

mit Aminen der Formel (VIII)

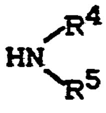

(VIII)

in der

$R^4$ und $R^5$ die vorstehend genannte Bedeutung haben,

gegebenenfalls in Gegenwart eines Lösungs- oder Verdünnungsmittels und gegebenenfalls in Gegenwart einer tertiären organischen Base, umsetzt.

Verwendet man zur Herstellung der Ausgangsverbindungen der Formel (II) beispielsweise 2,5-Bis-(cyclopentyloxycarbonyl)-3-methyl-4-hydroxythiophen und 2-Methoxyethylamin, so kann der Reaktionsverlauf durch das folgende Reaktionsschema wiedergegeben werden:

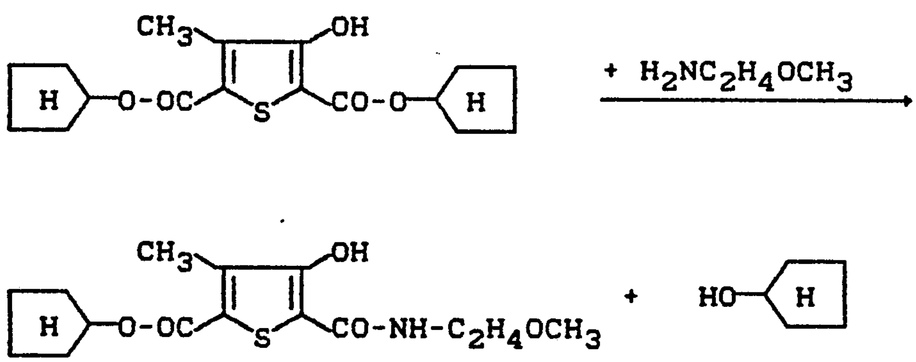


Ausgangsstoffe zur Herstellung von Verbindungen der Formel (II) sind beispielsweise 3-substituierte 2,5-Bis-(alkoxycarbonyl)-4-hydroxythiophene wie 3-Methyl-4-hydroxy-thiophen-2,5-dicarbonsäure-methyl-, -ethyl-, -isopropyl-, -1-methylpropyl-, -2,2-dimethylpropyl-, -cyanmethyl-, -2-cyanethyl-, -1-cyan-1-methyl-ethyl-, -2,2,2-trifluor=

ethyl-, -2-methoxyethyl-, -2-butylthioethyl-, -2-ethylthioethyl-, -allyl-, -methallyl-, -propargyl-, -1,1-dimethylpropargyl-, -cyclobutyl-, -cyclopentyl- und -cyclohexyl-ester; 4-Hydroxy-3-ethyl-, -3-propyl-, -3-isopropyl-, -3-butyl-, -3-tert.-butyl- und -3-phenyl-thiophen-2,5-dicarbonsäure-2,2,2-trifluor-ethylester.

Diese werden mit primären oder sekundären Aminen der Formel (VIII) umgesetzt wie Methylamin, Dimethylamin, Ethylamin, Diethylamin, n-Propylamin, Di-n-propylamin, iso-Propylamin, sek.-Butylamin, iso-Butylamin, n-Butylamin, Di-n-butylamin, Amylamin, N-Methylamylamin, 2-Methoxyethylamin, 2-Ethoxyethylamin, N-Methyl-2-methoxyethylamin, 3-Methoxypropylamin, 3-Butoxypropylamin, 2-Methylthioethylamin, 2-Butylthioethylamin, N-Methyl-3-butylthiopropylamin, 2-Cyanethylamin, 1-Cyan-1-methylethylamin, 5-Cyanpentylamin, 2,2-Difluorethylamin, 2,2,2-Trifluorethylamin, 3,3,3-Trifluorpropylamin, 2,2-Difluorbutylamin, 4,4,4-Trifluorbutylamin, 1-Trifluormethylethylamin, Allylamin, Diallylamin, Methallylamin, Propargylamin, N-Methylpropargylamin, Cyclopropylamin, Cyclobutylamin, Cyclopentylamin, Cyclohexylamin, N-Methylcyclohexylamin, N-Butoxyamin, N-Methyl-N-butoxyamin, Pyrrolidin, Piperidin, 2-, 3- oder 4-Methylpiperidin, 2-Ethylpiperidin, Morpholin, 2,6-Dimethylmorpholin, $N^1$-Methylpiperazin, $N^1$-Propylpiperazin, Thiazolidin und Hexahydro[1H]-azepin.

Als Verdünnungsmittel zur Herstellung der 2-alkoxycarbonylsubstituierten 4-Hydroxythiophen-5-carbonamide der Formel (II) kommen alle gegenüber den Reaktionspartnern inerten organische Lösungsmittel in Frage; vorzugsweise werden polare Lösungsmittel verwendet. Zu nennen sind hier beispielsweise: Acetonitril, Dimethylacetamid, Dimethylformamid, Dimethylsulfoxid, N-Methylpyrrolidon, Dioxan, Chlorbenzol, Benzonitril, Ethyldiisopropylamin, Triethylamin, Tributylamin, Dimethylcyclohexylamin, Ethyldicyclohexylamin, Dimethylbenzylamin, Pyridin, Picolin und Chinolin oder man verwendet das umzusetzende Amin der Formel (VIII) als Lösungsmittel.

Die Reaktionstemperaturen und die Reaktionsdauer werden durch die Aktivität der Ausgangsprodukte bestimmt. Im allgemeinen arbeitet man etwa zwischen 20 und 180$^0$C, vorzugsweise zwischen 40 und 150$^0$C.

Bei der Umsetzung niedrigsiedender Amine kann es von Vorteil sein, die Reaktion unter Druck vorzunehmen.

Je nach Arbeitsbedingungen fallen die 2-alkoxycarbonylsubstituierten 4-Hydroxythiophen-5-carbonamide der Formel (II) kristallin aus oder sie bleiben im organischen Lösungsmittel gelöst und können nach Abdestillieren überschüssigen Amins, welches aus wirtschaftlichen Gründen möglichst vollständig zurückgewonnen werden soll, und nach Waschen mit Wasser und verdünnter Säure isoliert werden, wobei sie gegebenenfalls durch Zugabe von wenig polaren Lösungsmitteln, wie Cyclohexan, Dibutylether oder Tetrachlorkohlenstoff aus ihren Lösungen abgeschieden werden.

Als Verdünnungsmittel für die erfindungsgemäße Umsetzung eines alkoxycarbonylsubstituierten 4-Hydroxythiophen-carbonamid-Derivats der Formel (II) mit einem Acylierungsmittel der Formel (III) nach Verfahrensvariante a) kommen alle gegenüber den Reaktionspartnern inerten organischen Lösungsmittel in Frage; vorzugsweise werden polare Lösungsmittel verwendet. Zu nennen sind hier beispielsweise Acetonitril, Aceton, Chloroform, Benzonitril, Dimethylacetamid, Dimethylformamid, Dimethylsulfoxid, Chlorbenzol, Essigsäureethylester, Dioxan, Methylethylketon, Methylenchlorid und Tetrahydrofuran.

Die Reaktionen können auch in heterogenen Systemen, bestehend aus Wasser und einem mit Wasser nicht mischbaren Lösungsmittel durchgeführt werden.

Als Säurebinder für die Umsetzung werden organische Basen verwendet, vorzugsweise tertiäre Amine. Zu nennen sind hier: Chinolin, Dimethylbenzylamin, Dimethylanilin, Ethyldicyclohexylamin, Ethyldiisopropylamin, Picolin, Pyridin und Triethylamin.

Als wasserbindende Agentien beim Einsatz von Carbonsäuren (Formel III: X = OH) werden bevorzugt Carbodiimide, wie z.B. Dicyclohexylcarbodiimid, verwendet.

Die Reaktionstemperaturen und die Reaktionsdauer werden durch die Aktivität der Ausgangsprodukte bestimmt. Im allgemeinen arbeitet man etwa zwischen -50 und +80°C, vorzugsweise zwischen -10 und +60°C.

Auch kann man zur Durchführung des erfindungsgemäßen Verfahrens Alkali- oder Erdalkalisalze des umzusetzenden Thiophenderivats in einem inerten Lösungsmittel vorlegen, oder man erzeugt das Salz, indem man in eine Mischung aus dem Thiophenderivat und einem hochsiedenden Lösungsmittel Alkalilauge, Alkoholate oder eine entsprechende Erdalkaliverbindung gibt und dann vorsichtig entwässert bzw. den Alkohol abdestilliert oder man gibt ein Alkali- oder Erdalkalihydrid hinzu.

Wird die Kondensation des Hydroxythiophenderivats mit einer Carbonsäure durch Wasserentzug mit einem Carbodiimid, beispielsweise Dicyclohexylcarbodiimid, durchgeführt, so läßt sich der entstehende schwerlösliche Harnstoff meist einfach von den leichtlöslichen erfindungsgemäßen Verbindungen abtrennen.

Als Verdünnungsmittel für die Umsetzung von alkoxycarbonylsubstituierten 4-Hydroxythiophen-carbonamiden der Formel (II) nach Verfahren b) mit Isocyanaten der Formel (IV) kommen alle gegenüber den Reaktionspartnern inerten organischen Lösungsmittel in Frage; vorzugsweise werden polare Lösungsmittel verwendet. Zu nennen sind hier beispielsweise Acetonitril, Aceton, Chloroform, Benzonitril, Dimethylacetamid, Dimethylformamid, Dimethylsulfoxid, Chlorbenzol, Essigsäureethylester, Dioxan, Methylethylketon, Methylenchlorid und Tetrahydrofuran.

Als Katalysatoren für die Umsetzung der Isocyanate mit den Hydroxythiophenen können tertiäre Amin, wie Triethylamin,

Triethylendiamin, Pyridin, 4-Dimethylaminopyridin oder auch Bleinaphthenat und Dibutylzinnoxid angewendet werden.

Die Reaktionstemperaturen und die Reaktionsdauer werden durch die Aktivität der Ausgangsprodukte bestimmt. Im allgemeinen arbeitet man zwischen 20 und 120° C, vorzugsweise zwischen 60 und 100° C.

Es kann erforderlich sein, einen Überschuß des Isocyanats der Formel (IV) in die Reaktion einzusetzen.

Zur Umsetzung eines alkoxycarbonylsubstituierten 4-Hydroxythiophencarbonamid-Derivats der Formel (II) erst mit Phosgen und anschließend mit einem Amin der Formel (VI) nach Verfahren c) wird in einer ersten Stufe eine Mischung bestehend aus einem 4-Hydroxythiophen-Derivat der Formel (II), einem tertiären Amin, wie z.B. Chinolin, Dimethylbenzylamin, Dimethylanilin, Ethyldicyclohexylamin, Ethyldiisopropylamin, Picolin, Pyridin oder Triethylamin, und einem inerten organischen Lösungsmittel, wie z.B. Methylenchlorid, Chloroform, Chlorbenzol, Ethylacetat, Toluol oder Xylol, zu überschüssigem Phosgen, gelöst in dem inerten organischen Lösungsmittel, gegeben. Dabei arbeitet man im allgemeinen zwischen -50 und +80° C, vorzugsweise bei 0 bis +30° C.

Nach Isolierung des 4-Chlorcarbonyloxythiophen-Derivats der Formel (V) wird dieses mit einem Amin der Formel (VI) oder mit einem Salz des Amins der Formel (VI) umgesetzt, wobei man aus wirtschaftlichen Gründen die entstehende

Säure an einem tertiären Amin bindet. Wird ein Aminsalz in die Reaktion eingeführt, ist die doppelte Menge des tertiären Amins einzusetzen. Für diese Umsetzung kommen im Prinzip mit Wasser mischbare oder nicht mischbare Lösungsmittel, wie vorstehend aufgeführt, in Frage. Im allgemeinen arbeitet man bei Temperaturen entsprechend der Phosgenierungsreaktion.

Je nach Arbeitsbedingungen fallen die erfindungsgemäßen Wirkstoffe kristallin aus oder sie bleiben im organischen Lösungsmittel gelöst und können dann nach Auswaschen der Lösung mit Wasser durch vorsichtiges Einengen der Lösung oder durch Zugabe wenig polarer organischer Lösungsmittel, wie Cyclohexan, Dibutylether, Diisopropylether oder Tetrachlorkohlenstoff, abgeschieden werden. Gegebenenfalls müssen mit Wasser mischbare polare Lösungsmittel nach der Reaktion durch Abdampfen im Vakuum entfernt werden.

Sind die erfindungsgemäßen Verbindungen in einem mit Wasser mischbaren Lösungsmittel gelöst, so können sie auch durch Zugabe von Wasser ausgefällt werden.

Die erfindungsgemäßen Verbindungen zersetzen sich zum Teil bei höherer Temperatur; in diesen Fällen können die Schmelzpunkte nur mit geringer Genauigkeit oder überhaupt nicht ermittelt werden. Das Vorliegen bestimmter Strukturelemente ist aus den NMR-Spektren ersichtlich.

Die erfindungsgemäßen Wirkstoffe weisen eine starke biologische Wirkung auf und können zur Bekämpfung von Schädlingen praktisch eingesetzt werden. Die Wirkstoffe sind somit u.a. für den Gebrauch als Pflanzenschutzmittel, insbesondere als Fungizide, geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;

Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie

Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Die erfindungsgemäßen Verbindungen der Formel (I) sind besonders gut wirksam gegen Pilzkrankheiten im Reis, sie zeigen aber auch bei entsprechenden Anwendungskonzentrattionen gute Wirksamkeit gegen Oomyceten, den Erregern des Apfelschorfs, gegen Botrytis-Erreger.

Einige der Wirkstoffe eignen sich bei guter Pflanzenverträglichkeit und günstiger Warmblütertoxizität u.a. auch zur Bekämpfung von tierischen Schädlingen, wie Insekten und Spinnentieren, die in der Landwirtschaft, in Forsten, im Vorrats- und Materialschutz sowie auf dem Hygienesektor vorkommen, insbesondere gegen Insekten. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.

Aus der Ordnung der Collembola z.B. Onychiurus armatus. Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermoptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Herstellungsbeispiele

Beispiel 1:

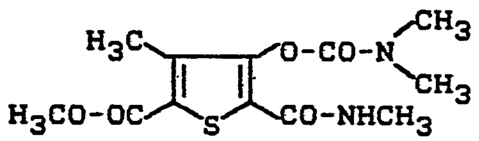

Zu einer Suspension, bestehend aus 10 g 2-Methoxycarbonyl-3-methyl-4-hydroxythiophen-5-carbonsäuremethylamid, 40 ml Pyridin und 10 mg Dimethylaminopyridin werden 5,9 g Dimethylcarbamidsäurechlorid getropft. Man rührt 2 h bei 45°C und 21 h bei 22°C. Der ausgefallene kristalline Stoff wird abgetrennt, mit Wasser chloridfrei gewaschen und bei 60°C/0,1 mbar getrocknet. Man erhält 6,9 g 2-Methoxycarbonyl-3-methyl-4-(dimethylaminocarbonyloxy)-thiophen-5-carbonsäure-methylamid mit einem Schmelzpunkt von 173°C. Aus der Mutterlauge kann noch eine Produktfraktion von 3,2 g gewonnen werden.

Herstellung des Vorproduktes

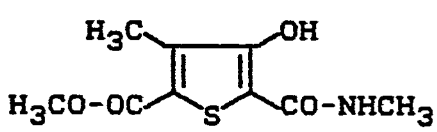

20 g 2,5-Bis-(methoxycarbonyl)-3-methyl-4-hydroxythiophen werden in 110 ml Dimethylformamid gelöst. Es wird Methylamin eingeleitet. Zunächst entsteht das Methylamin-Salz des 4-Hydroxythiophen Derivates, welches schwerlöslich ist. Man erhitzt unter weiterem Einleiten von Methylamin 5 h auf 60° C und 3 h auf 80° C. Es wird im Vakuum eingedampft. Der Abdampfrückstand wird mit 300 ml Ethylacetat und 100 ml eiskalter 5 %iger Schwefelsäure behandelt. Die entstehenden Kristalle werden abgetrennt, mit Wasser gewaschen und bei 60° C/0,1 mbar getrocknet.
Ausbeute: 11,6 g 2-Methoxycarbonyl-3-methyl-4-hydroxythiophen-5-carbonsäuremethylamid mit einem Schmelzpunkt von 188° C (Zersetzung).

Beispiel 2:

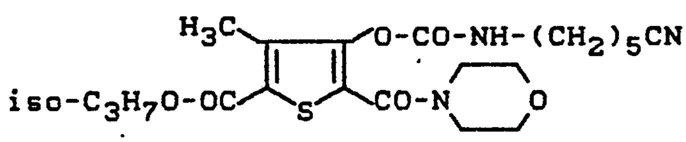

9 g 2-(Isopropoxycarbonyl)-3-methyl-4-hydroxythiophen-5-carbonsäuremorpholid, 5 ml Acetonitril, 5 g 5-Cyanpentylisocyanat und 100 mg Triethylendiamin werden 15 h auf 100° C gehalten. Man verdünnt mit 50 ml Toluol, behandelt die Reaktionsmischung mit 1 g Kieselgel und dampft im Vakuum ein. Der Abdampfrückstand wird bei 60° C/0,1 mbar getrocknet. Nach Behandlung mit Diisopropylether kristallisiert das Produkt. Die Kristalle werden abgetrennt, mit Diisopropylether gewaschen und bei 60° C/0,1 mbar getrocknet.
Ausbeute: 12,1 g 2-(Isopropoxycarbonyl)-3-methyl-4-(5-cyanpentylaminocarbonyloxy)-thiophen-5-carbonsäure-morpholid mit einem Schmelzpunkt von 81° C.

Herstellung des Vorproduktes:

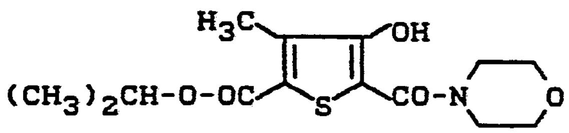

90 g 2,5-Bis-(isopropoxycarbonyl)-3-methyl-4-hydroxythiophen und 450 g Morpholin werden 9 1/2 h auf 124°C erhitzt. Das überschüssige Morpholin wird im Vakuum abgedampft. Der Abdampfrückstand wird in 600 ml Dichlormethan gelöst, zweimal mit eiskalter, verdünnter Schwefelsäure und einmal mit Wasser gewaschen. Die Lösung wird mit Natriumsulfat getrocknet. Nach Abdampfen des Lösungsmittels wird der Rückstand mit wenig Diisopropylether angerieben, abgetrennt und bei 60°C/0,1 mbar getrocknet.
Ausbeute: 78,9 g 2-Isopropoxycarbonyl-3-methyl-4-hydroxy-5-(1-aza-4-oxacyclohex-1-ylcarbonyl)-thiophen mit einem Schmelzpunkt von 123°C.

Auf gleiche Weise werden die Verbindungen der Formel (I) gewonnen:

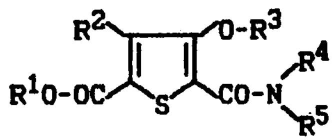

| Bsp. Nr. | Herst. gem. Bsp. | $R^1$ | $R^2$ | $R^3$ | $R^4$ | $R^5$ | Physikalische Daten [F = Schmelzpunkt °C] |
|---|---|---|---|---|---|---|---|
| 3 | 2 | $H_3C$- | $H_3C$- | $-OC-NH(CH_2)_5CN$ | $-CH_3$ | -H | F 128-146 Rohprodukt |
| 4 | 2 | $H_3C$- | $(CH_3)_2CH$- | $-OC-NH-C_6H_4-CH_3$(m) | $-C_2H_4-O-C_2H_4-$ | | F 154 aus Acetonitril |
| 5 | 2 | $H_5C_2$- | $H_3C$ | $-OC-NH-CH_3$ | $-(CH_2)_5-$ | | F 118 aus Diisopropylether |
| 6 | 1 | $H_5C_2$ | $H_3C$- | $-OC-N(CH_3)_2$ | $-(CH_2)_5-$ | | zäh viskos |
| 7 | 2 | $n-H_7C_3$- | $H_3C$- | $-OC-NH-C_6H_4-CF_3$(m) | $-(CH_2)_5-$ | | |
| 8 | 2 | $n-H_7C_3$- | $H_3C$- | $-OC-NH-C_6H_4-CH_3$(m) | $-(CH_2)_5-$ | | F 134 aus Acetonitril |
| 9 | 2 | $(CH_3)_2CH$- | $H_3C$- | $-OC-NH-C_6H_4-OCF_3$(m) | $-C_2H_4-O-C_2H_4-$ | | F 165 aus Acetonitril |
| 10 | 1 | " | " | $-SO_2-CH_2Cl$ | $-(CH_2)_5-$ | | F 85 aus Petrolether |
| 11 | 2 | " | " | $-OC-NH-C_6H_4-CH_3$(m) | $-(CH_2)_4-$ | | F 124 aus Acetonitril |
| 12 | 1 | " | " | $-OC-C_6H_4-CH_3$(m) | $-(CH_2)_5-CN$ | -H | F 79 aus Diisopropylether |
| 13 | 1 | " | " | $-OC-C_6H_4-CH_3$(m) | $-C_2H_4-O-C_2H_4-$ | | F 124 aus Petrolether |
| 14 | 2 | " | " | $-OC-NH-CH_3$ | $-C_2H_4-O-C_2H_4-$ | | F 130 aus Diisopropylether |
| 15 | 2 | " | " | $-OC-NH-CH_3$ | $-(CH_2)_5-$ | | F 142 aus Acetonitril |
| 16 | 2 | " | " | $-OC-NH-(CH_2)_5-CN$ | $-(CH_2)_5-$ | | F 78 aus Acetonitril |
| 17 | 1 | " | " | $-OC-N(CH_3)_2$ | $-C_2H_4-O-C_2H_4-$ | | F 112 aus Diisopropylether |

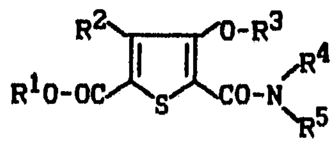

| Bsp. Nr. | Herst. gem. Bsp. | $R^1$ | $R^2$ | $R^3$ | $R^4$ | $R^5$ | Physikalische Daten [F = Schmelzpunkt °C] |
|---|---|---|---|---|---|---|---|
| 18 | 2 | $(H_3C)_2CH-$ | $H_3C-$ | $-OC-NH-C_6H_4-CF_3$(m) | $-C_2H_4-O-C_2H_4-$ | | F 165 aus Acetonitril |
| 19 | 2 | " | " | $-OC-NH-(CH_2)_5-COOC_4H_9$ | $-C_2H_4-O-C_2H_4-$ | | F 81 aus Ligroin |
| 20 | 2 | " | " | $-OC-NH-(CH_2)_5-COOCH_2C(CH_3)_3$ | $-C_2H_4-O-C_2H_4-$ | | F 57 aus Petrolether |
| 21 | 2 | " | " | $-OC-NH-C_6H_4-CH_3$(m) | $-CH_2)_5-$ | | zäh viskos |
| 22 | 1 | " | $C_6H_5-$ | $-OC-C_6H_5$ | $-C_2H_4-O-C_2H_4-$ | | F 131 aus Toluol/ Diisopropylether |
| 23 | 2 | $H_5C_2(H_3C)CH-$ | $H_3C$ | $-OC-NH(CH_2)_5-CN$ | $-C_2H_4-O-C_2H_4-$ | | zäh viskos |
| 24 | 2 | " | " | $-OC-NH-C_6H_5-CH_3$(m) | $-CH_2)_5-$ | | F 150 aus Diethylketon |
| 25 | 2 | " | " | $-OC-NH-C_6H_4-CF_3$(m) | $-(CH_2)_5-$ | | F 141 aus Diethylketon |
| 26 | 2 | " | " | $-OC-NH-C_6H_4-Cl$ (p) | $-(CH_2)_5-$ | | F 157 aus Aceton |
| 27 | 2 | " | " | $-OC-NH-(CH_2)_5-CN$ | $-(CH_2)_5-$ | | zäh viskos |
| 28 | 2 | " | " | $-OC-NH-C_6H_4-CH_3$(o) | $-C_2H_4-O-C_2H_4-$ | | F 114 aus Diispropylether |
| 29 | 2 | $H_3C-$ | $H_3C-$ | $-OC-NH-CH_3$ | $-CH_3$ | -H | F Zersetzung >100° |

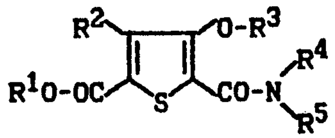

| Bsp. Nr. | Herst. gem. Bsp. | $R^1$ | $R^2$ | $R^3$ | $R^4$ | $R^5$ | Physikalische Daten [F = Schmelzpunkt °C] |
|---|---|---|---|---|---|---|---|
| 30 | 1 | $H_3C$- | $H_3C$- | $-OC-C_6H_4-F(p)$ | $-CH_3$ | -H | 187 aus Diethylketon |
| 31 | 2 | $H_5C_2-(H_3C)CH$- | " | $-OC-NH-C_6H_4-CF_3(m)$ | $-C_2H_4-O-C_2H_4-$ | | 153 aus Acetonitril |
| 32 | 2 | $H_5C_2$- | " | $-OC-NH-C_6H_4-CH_3(o)$ | $-C_2H_4-O-C_2H_4-$ | | 116 aus " |
| 33 | 2 | $(H_3C)_2CH$- | " | $-OC-NH-C_6H_4-CF_3(m)$ | $-C_5H_{10}-$ | | 156 aus " |
| 34 | 2 | $H_7C_3$- | " | $-OC-NH-C_6H_4-CH_3(m)$ | $-C_2H_4-O-C_2H_4-$ | | 148,5 aus " |
| 35 | 2 | $H_7C_3$- | " | $-OC-NH-C_6H_4-CF_3(m)$ | $-C_2H_4-O-C_2H_4-$ | | 137 aus " |

Anwendungsbeispiele:

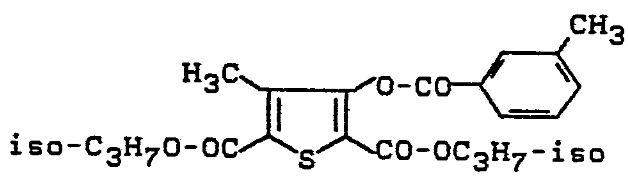

Beispiel

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß den Herstellungsbeispielen: 2, 16, 23 und 20.

Patentansprüche

1. Acyloxythiophen-carbonamide der allgemeinen Formel (I)

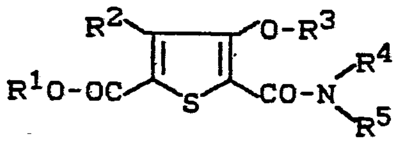

(I)

in welcher

$R^1$ für Alkyl, Alkoxyalkyl, Alkylthioalkyl, Fluoralkyl, Cyanalkyl, Alkenyl, Alkinyl oder für Cycloalkyl steht,

$R^2$ für Alkyl oder gegebenenfalls substituiertes Phenyl steht,

$R^3$ für Alkylsulfonyl, Halogenalkylsulfonyl, für gegebenenfalls substituiertes Phenylsulfonyl oder für den Rest -OC-Q steht, in dem

Q für Alkyl steht, das gegebenenfalls gleich oder verschieden durch Halogen, Alkoxy, Alkylthio, Alkylsulfonyl, Alkylthioalkoxy, Alkoxycarbonyl, Phenyl, Phenoxy und Halogenphenoxy substituiert ist; für Alkenyl, das gegebenenfalls gleich oder verschieden durch Halogen, Phenyl, Halogenphenyl und Halogenalkylphenyl substituiert ist; für Alkinyl; für Cycloalkyl; für Alkoxy, das gegebenenfalls substituiert ist durch Halogen oder Cyan; für Phenyl, das gegebenenfalls gleich oder verschieden substituiert ist durch Halogen, Alkyl, Halogenalkyl, Alkoxy, Halogenalkoxy, Alkylthio, Halogenalkylthio, Alkylsulfinyl, Halogenalkylsulfinyl, Alkylsulfonyl und Halogenalkylsulfonyl; für Pyridyl; für Monoalkylamino, wobei der Alkylteil gegebenenfalls substituiert ist durch Halogen, Cyan, Alkoxycarbonyl, Dialkylaminocarbonyl, Alkanylenaminocarbonyl oder Oxaalkanylenaminocarbonyl; für 1-Cyancycloalkylamino; für Dialkylamino; für Alkanylenamino; für Oxaalkanylenamino; für N-Alkyl-N-phenylamino oder Phenylamino steht, worin jeweils der Phenylrest gegebenenfalls gleich oder verschieden substituiert ist durch Halogen, Alkyl, Halogenalkyl, Alkoxy, Halogenalkoxy, Alkylthio, Halogenalkylthio, Alkylsulfonyl, Halogenalkylsulfonyl, Alkanylendioxy, Halogenalkanylendioxy oder durch $-O-CHal_2-O-CHal_2-$oder für Alkoxycarbonylamino steht,

$R^4$ für Alkyl, Alkoxyalkyl, Alkylthioalkyl, Cyanalkyl, Fluoralkyl, Alkenyl, Alkinyl, Cycloalkyl oder Alkoxy steht,

$R^5$ für Wasserstoff, Alkyl, Alkoxyalkyl, Alkylthioalkyl, Fluoralkyl, Cyanalkyl, Alkenyl, Alkinyl oder Cycloalkyl steht,

oder

$R^4$ und $R^5$ gemeinsam mit dem Stickstoffatom, an dem sie stehen, für einen gegebenenfalls substituierten Heterocyclus stehen, welcher in der Alkylenkette durch weitere Heteroatome substituiert sein kann.

2. Acyloxythiophen-carbonamide gemäß Anspruch 1, worin in der allgemeinen Formel (I)

$R^1$ für Alkyl mit 1 bis 5 Kohlenstoffatomen, für Alkoxyalkyl oder für Alkylthioalkyl mit je 1 bis 5 Kohlenstoffatomen je Alkylteil, für Fluoralkyl mit 1 bis 5 Kohlenstoffatomen und 1 bis 5 Fluoratomen, für Cyanalkyl mit 1 bis 5 Kohlenstoffatomen im Alkylteil, für Alkenyl mit 3 oder 4 Kohlenstoffatomen, für Alkinyl mit 3 bis 5 Kohlenstoffatomen oder für Cycloalkyl mit 4 bis 6 Kohlenstoffatomen steht,

$R^2$ für Alkyl mit 1 bis 4 Kohlenstoffatomen oder für gegebenenfalls ein- bis fünffach, gleich oder verschieden substituiertes Phenyl steht, wobei als Substituenten Alkyl mit 1 bis 4 Kohlenstoffatomen, Alkoxy oder Alkylthio mit je 1 bis 4 Kohlenstoffatomen, Halogen, Nitro, Halogenalkyl, Halogenalkoxy oder Halogenalkylthio mit je 1 bis 4 Kohlenstoffatomen und 1 bis 5 gleichen oder verschiedenen Halogenatomen in Frage kommen,

$R^3$ für Alkylsulfonyl mit 1 bis 4 Kohlenstoffatomen, für Halogenalkylsulfonyl mit 1 bis 4 Kohlenstoffatomen und 1 bis 4 gleichen oder verschiedenen Halogenatomen, für Phenylsulfonyl steht, wobei der Phenylrest 1- bis 5-fach, gleich oder verschieden durch Halogen, Alkyl mit 1 bis 4 Kohlenstoffatomen oder Nitro substituiert sein kann oder für den Rest -OC-Q steht,
in dem

Q für Alkyl mit 1 bis 4 Kohlenstoffatomen steht und gegebenenfalls durch bis zu 4 gleichen oder verschiedenen Halogenatomen, Alkoxy, Alkylthio, Alkylsulfonyl, Alkoxycarbonyl mit je bis zu 4 Kohlenstoffatomen, Alkylthioalkoxy mit je bis zu 2 Kohlenstoffatomen je Alkyleinheit, Phenyl, Phenoxy oder Halogenphenoxy mit bis zu 3 gleichen oder verschiedenen Halogenatomen substituiert sein kann; für Alkenyl mit 2 bis 4 Kohlenstoffatomen steht und gegebenenfalls substituiert sein kann durch 1 bis 4 gleiche oder verschiedene Halogenatome, Phenyl, Halogenphenyl mit 1 bis 3 gleichen oder verschiedenen Halogenatomen oder Halogenalkylphenyl mit 1 bis 3 gleichen oder verschiedenen Halogenatomen und 1 oder 2 Kohlenstoffatomen im Halogenalkylrest; für Alkinyl mit bis zu 3 Kohlenstoffatomen; für Cycloalkyl mit 3 bis 6 Kohlenstoffatomen; für Alkoxy mit 1 bis 4 Kohlenstoffatomen, das gegebenenfalls substituiert sein kann mit bis zu 3 gleichen oder verschiedenen Halogenatomen oder Cyan; für Phenyl, das gegebenenfalls gleich oder verschieden, ein- bis dreifach substituiert sein kann durch gleiche oder verschiedene Halogenatome, Alkyl mit 1 bis 4 Kohlenstoffatomen, Halogenalkyl mit 1 oder 2 Kohlenstoffatomen und 1 bis 3 gleichen oder verschiedenen Halogenatomen, Alkoxy mit 1 oder 2 Kohlenstoffatomen, Halogenalkoxy mit 1 oder 2 Kohlenstoffatomen und 1 bis 3 gleichen oder verschiedenen Halogenatomen, Alkylthio mit 1 oder 2 Kohlenstoffatomen, Halogenalkylthio mit 1 oder 2 Kohlenstoffatomen und 1 bis 3 gleichen oder verschiedenen Halogenatomen, Alkylsulfinyl mit 1 oder 2 Kohlenstoffatomen, Halogenalkylsulfinyl mit 1 oder 2 Kohlenstoffatomen und 1 bis 3 gleichen oder verschiedenen Halogenatomen, Alkylsulfonyl mit 1 oder 2 Kohlenstoffatomen oder Halogenalkylsulfonyl mit 1 oder 2 Kohlenstoffatomen und 1 bis 3 gleichen oder verschiedenen Halogenatomen; für Pyridyl; für Alkylamino mit 1 bis 6 Kohlenstoffatomen, das gegebenenfalls in der Alkylgruppe mit 1 bis 4 gleichen oder verschiedenen Halogenatomen, Cyan, Alkoxycarbonyl mit 1 bis 6 Kohlenstoffatomen in der Alkoxygruppe, Dialkylaminocarbonyl mit je 1 bis 3 Kohlenstoffatomen je Alkylteil, Alkanylenaminocarbonyl oder Oxaalkanylenaminocarbonyl mit je bis zu 6 Kohlenstoffatomen im Alkanylenteil sein kann; für 1-Cyancycloalkylamino mit 5 oder 6 Kohlenstoffatomen im Cycloalkylteil; für Dialkylamino mit 1 bis 3 Kohlenstoffatomen je Alkylteil, Alkanylenamino und Oxaalkanylenamino mit je bis zu 6 Kohlenstoffatomen, N-Alkyl-N-phenylamino mit 1 bis 3 Kohlenstoffatomen im Alkylteil oder für Phenylamino, wobei jeweils der Phenylrest gleich oder verschieden, ein- bis dreifach substituiert sein kann durch gleiche oder verschiedene Halogenatome, durch Alkyl, Alkoxy, Alkylthio und Alkylsulfonyl mit je 1 oder 2 Kohlenstoffatomen,

Halogenalkyl, Halogenalkoxy, Halogenalkylthio und Halogenalkylsulfonyl mit je 1 oder 2 Kohlenstoffatomen und 1 bis 3 gleichen oder verschiedenen Halogenatomen, Alkanylendioxy mit 1 oder 2 Kohlenstoffatomen, Halogenalkanylendioxy mit 1 oder 2 Kohlenstoffatomen und bis zu 4 Halogenatomen oder durch den Rest $-O-CHal_2-O-CHal_2-$ oder für Alkoxycarbonylamino mit 1 bis 8 Kohlenstoffatomen im Alkylteil steht,

$R^4$ für Alkyl mit 1 bis 5 Kohlenstoffatomen, für Alkoxyalkyl oder Alkylthioalkyl mit jeweils 1 bis 5 Kohlenstoffatomen je Alkylteil, für Cyanalkyl mit 1 bis 5 Kohlenstoffatomen im Alkylteil, für Fluoralkyl mit 1 bis 3 Fluoratomen und 1 bis 5 Kohlenstoffatomen, für Alkenyl oder Alkinyl mit je 3 bis 5 Kohlenstoffatomen, für Cycloalkyl mit 3 bis 6 Kohlenstoffatomen oder für Alkoxy mit 1 bis 5 Kohlenstoffatomen steht,

$R^5$ für Wasserstoff, Alkyl mit 1 bis 5 Kohlenstoffatomen, für Alkoxyalkyl oder Alkylthioalkyl mit jeweils 1 bis 5 Kohlenstoffatomen je Alkylteil, für Cyanalkyl mit 1 bis 5 Kohlenstoffatomen im Alkylteil, für Fluoralkyl mit 1 bis 3 Fluoratomen und 1 bis 5 Kohlenstoffatomen, für Alkenyl oder Alkinyl mit 3 bis 5 Kohlenstoffatomen, für Cycloalkyl mit 6 Kohlenstoffatomen steht, oder

$R^4$ und $R^5$ zusammen mit dem Stickstoffatom für einen 5-, 6- oder 7-gliedrigen heterocyclischen Ring stehen, der Aza-, Oxa- oder Thiaelemente enthalten und durch Alkylgruppen mit 1 bis 4 Kohlenstoffatomen gleich oder verschieden substituiert sein kann.

3. Acyloxythiophen-carbonamide gemäß Anspruch 1, worin in der allgemeinen Formel (I)

$R^1$ für Methyl, Ethyl, n- oder iso-Propyl, 2,2-Dimethylpropyl, 2-Methoxyethyl, 2-Ethoxyethyl, 2-Methylthioethyl, 2-Ethylthioethyl, 2,2,2-Trifluorethyl, Cyanmethyl, Cyanethyl, Allyl, Methallyl, 2-Propinyl, 1,1-Dimethyl-2-propinyl, Cyclopentyl oder Cyclohexyl steht,

$R^2$ für Methyl, Ethyl, n-Propyl, iso-Propyl, n-Butyl, sek.-Butyl, iso-Butyl oder tert.-Butyl, Phenyl oder 1- bis 3-fach, gleich oder verschieden durch Halogen substituiertes Phenyl steht,

$R^3$ für Methansulfonyl, Chlormethansulfonyl, Phenylsulfonyl, 2-, 3- und 4-Chlorphenylsulfonyl, 3,4-Dichlorphenylsulfonyl, 2- und 4-Methylphenylsulfonyl, 3-Chlor-4-methylphenylsulfonyl, 2-, 3- und 4-Nitrophenylsulfonyl, 2-Chlor-5-nitrophenylsulfonyl, 2,4,5-Trifluor-3-nitrophenylsulfonyl, 2-Methyl-5-nitrophenylsulfonyl oder für den Rest -OC-Q steht, wobei

Q für Methyl, Ethyl, Isopropyl, Butyl, Dichlormethyl, Trichlormethyl, Trifluormethyl, 1-Brom-1-methyl-ethyl, Methoxymethyl, Butoxymethyl, 2-Butylthioethyl, Propylsulfonylmethyl, Methoxycarbonylethyl, Butoxycarbonylethyl, Methylthioethoxymethyl, Benzyl, Phenoxymethyl, 1-Phenoxyethyl, 1-(4-Chlorphenoxy)-ethyl, 2,4-Dichlorphenoxymethyl; Vinyl, Propen-1-yl, 2-Chlorvinyl, 2-(Phenyl)-vinyl, 2-(4-Chlorphenyl)-vinyl, 2-(4-Chlorphenyl)-2-chlorvinyl, 2-(2-Trifluormethylphenyl)-vinyl, Ethinyl, 2-Methyl-ethinyl, Cyclopropyl, Cyclobutyl, Cyclopentyl, Cyclohexyl, Methoxy, Isopropoxy, Butoxy, 2-Chlorethoxy, 2,2,2-Trichlorethoxy, 2,2,2-Trifluorethoxy, 2-Cyanethoxy; Phenyl, 2-, 3- oder 4-Chlorphenyl, 2-Bromphenyl, 4-Fluorphenyl, 2-, 3- oder 4-Methylphenyl, 4-tert.-Butylphenyl, 2-, 3- oder 4-Trifluormethylphenyl, 3-Chlor-4-trifluormethylphenyl, 4-Methoxyphenyl, 3-Trifluormethoxyphenyl, 3-Chlor-4-trifluormethoxyphenyl, 4-Chlordifluormethoxyphenyl, 4-Ethylthiophenyl, 4-Trifluormethylthiophenyl, 4-Ethylsulfinylphenyl, 4-Trifluormethylsulfinylphenyl, 4-Methylsulfonylphenyl, 4-Trifluormethylsulfonylphenyl; 2-, 3- oder 4-Pyridyl; Methylamino, Butylamino, Hexylamino, 2,2,2-Trifluorethylamino, 1-Trifluormethyl-ethylamino, 6-Chlorhexylamino, 2-Cyanethylamino, 1-Cyan-1-methylethylamino, 1-Cyan-1-methylpropylamino, 1-Cyano-1-ethylpropylamino, 1-Cyan-cyclopent-1-ylamino,

1-Cyan-cyclohex-1-ylamino, 3-Cyanpropylamino, 5-Cyanpentylamino, 6-Cyanhexylamino, Methoxycarbonylmethylamino, Ethoxycarbonylmethylamino, Butylcarbonylethylamino, Isobutoxycarbonylethylamino, 1-Methoxycarbonyl-1-methyl-ethylamino, 1-Propoxycarbonyl-1-methyl-ethylamino, 1-Ethoxycarbonyl-1-ethyl-ethylamino, 1-Isobutoxycarbonyl-1-ethyl-ethylamino, Methoxycarbonylpropylamino, Methoxycarbonyl-pentylamino, Isopropoxycarbonyl-pentylamino, sek.-Butyloxycarbonylpentylamino, 2-Ethoxycarbonyl-2-ethyl-butylamino, Butoxycarbonylpentylamino, 5-(2,2-Dimethylpropyloxycarbonyl)-pentylamino, N-Morpholinocarbonylmethylamino, 1-(N,N-Diethylaminocarbonyl)-ethylamino, 2-(N-Pyrrolidinocarbonyl)-ethylamino, 3-(N-Piperidinocarbonyl)-propylamino, 5-(N,N-Dimethylaminocarbonyl)-pentylamino; 1-Cyancyclohex-1-ylamino, Dimethylamino, Diethylamino, Pyrrolidino, Piperidino, 3,5-Dimethylmorpholino, N-Methyl-N-phenylamino, Phenylamino, 2-Chlorphenylamino, 2,4,5-Trichlorphenylamino, 4-Fluorphenylamino, 2-, 3- oder 4-Methylphenylamino, 3,5-Dimethylphenylamino, 2-, 3- oder 4-Trifluormethylphenylamino, 2-Chlor-5-trifluormethylphenylamino, 4-Ethoxyphenylamino, 3,5-Dichlor-4-methoxyphenylamino, 4-Trifluormethoxyphenylamino, 4-Ethylthiophenylamino, 3-Trifluormethylthiophenylamino, 4-Ethylsulfonylphenylamino, 3-Trifluormethylsulfonylphenylamino, 3,4-Methylendioxyphenylamino, 3,4-Difluormethylendioxyphenylamino, 3,4-(Tetrafluorethylendioxy)-phenylamino, 2,2,4,4-Tetrafluorbenzodiox-1,3-en-6-ylamino; Methoxycarbonylamino, Ethoxycarbonylamino, Butoxycarbonylamino und 2-Ethylhexoxycarbonylamino steht,

$R^4$ für Methyl, Ethyl, n- oder iso-Propyl, 2,2-Dimethylpropyl, 2-Methoxyethyl, 2-Ethoxyethyl, 2-Propoxyethyl, 3-Methoxypropyl, 3-Ethoxypropyl, 3-Butoxypropyl, 2-Methylthioethyl, 2-Ethylthioethyl, 2,2,2-Trifluorethyl, 2-Cyanethyl, 1-Methyl-1-cyanoethyl, ω-Cyanpentyl, Allyl, Methallyl, 2-Propinyl, 1,1-Dimethyl-2-propinyl, Cyclopropyl, Cyclobutyl, Cyclopentyl, Cyclohexyl, Methoxy, Ethoxy, n-Propyloxy, n-Butyloxy, sek.-Butyloxy oder n-Pentyloxy steht,

$R^5$ für Wasserstoff, Methyl, Ethyl, n- oder iso-Propyl, 2,2-Dimethylpropyl, 2-Methoxyethyl, 2-Ethoxyethyl, 2-Propoxyethyl, 3-Methoxypropyl, 3-Ethoxypropyl, 3-Butoxypropyl, 2-Methylthioethyl, 2-Ethylthioethyl, 2,2,2-Trifluorethyl, 2-Cyanethyl, 1-Methyl-1-cyano-ethyl, ω-Cyanpentyl, Allyl, Methallyl, 2-Propinyl, 1,1-Dimethyl-2-propinyl oder Cyclohexyl steht, oder

$R^4$ und $R^5$ zusammen mit dem Stickstoffatom für Pyrrolidin, Piperidin, 2-Methylpiperidin, 3-Methylpiperidin, 4-Methylpiperidin, Hexahydro-1H-azepin, Morpholin, 2,6-Dimethylmorpholin, Thiazolidin, $N^1$-Methylpiperazin oder $N^1$-Propylpiperazin stehen.

4. Acyloxythiophen-carbonamide gemäß Anspruch 1, wobei in der allgemeinen Formel (I)

$R^1$ für Methyl, Ethyl, Isopropyl, n-Propyl, sek.-Butyl, Cyclopentyl oder Cyclohexyl steht,

$R^2$ für Methyl, Ethyl, Isopropyl, tert.-Butyl oder Phenyl steht,

$R^3$ für Methansulfonyl, Chlormethansulfonyl, Chlorethansulfonyl, p-Toluylsulfonyl, Benzoyl, 3-Methyl-, 4-Fluor-, 3-Trifluormethylbenzoyl; Methylaminocarbonyl, N,N-Dimethyl-, 2-Methyl-2-cyanethyl-, 5-Cyanpentyl-, 5-Butoxycarbonylpentyl-, 5-(2,2-Dimethylpropoxycarbonyl)-pentylaminocarbonyl; 2-, 3- und 4-Toluylaminocarbonyl; 3-Trifluormethyl-, 3-Trifluormethoxyphenyl-aminocarbonyl oder 2-Ethylhexoxycarbonyl-aminocarbonyl steht,

$R^4$ für Methyl, Methoxyethyl, Methoxypropyl oder Cyanpentyl steht und

$R^5$ für Wasserstoff steht oder

$R^4$ und $R^5$ zusammen für Butanylen, Pentanylen oder 3-Oxapentanylen stehen.

5. Verfahren zur Herstellung von Acyloxythiophen-carbonamiden der allgemeinen Formel (I)

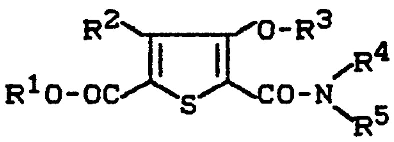

(I)

in welcher

$R^1$ für Alkyl, Alkoxyalkyl, Alkylthioalkyl, Fluoralkyl, Cyanalkyl, Alkenyl, Alkinyl oder für Cycloalkyl steht,

$R^2$ für Alkyl oder gegebenenfalls substituiertes Phenyl steht,

$R^3$ für Alkylsulfonyl, Halogenalkylsulfonyl, für gegebenenfalls substituiertes Phenylsulfonyl oder für den Rest -OC-Q steht, in dem

Q für Alkyl steht, das gegebenenfalls gleich oder verschieden durch Halogen, Alkoxy, Alkylthio, Alkylsulfonyl, Alkylthioalkoxy, Alkoxycarbonyl, Phenyl, Phenoxy und Halogenphenoxy substituiert ist; für Alkenyl, das gegebenenfalls gleich oder verschieden durch Halogen, Phenyl, Halogenphenyl und Halogenalkylphenyl substituiert ist; für Alkinyl; für Cycloalkyl; für Alkoxy, das gegebenenfalls substituiert ist durch Halogen oder Cyan; für Phenyl, das gegebenenfalls gleich oder verschieden substituiert ist durch Halogen, Alkyl, Halogenalkyl, Alkoxy, Halogenalkoxy, Alkylthio, Halogenalkylthio, Alkylsulfinyl, Halogenalkylsulfinyl, Alkylsulfonyl und Halogenalkylsulfonyl; für Pyridyl; für Monoalkylamino, wobei der Alkylteil gegebenenfalls substituiert ist durch Halogen, Cyan, Alkoxycarbonyl, Dialkylaminocarbonyl, Alkanylenaminocarbonyl oder Oxaalkanylenaminocarbonyl; für 1-Cyancycloalkylamino; für Dialkylamino; für Alkanylenamino; für Oxaalkanylenamino; für N-Alkyl-N-phenylamino oder Phenylamino steht, worin jeweils der Phenylrest gegebenenfalls gleich oder verschieden substituiert ist durch Halogen, Alkyl, Halogenalkyl, Alkoxy, Halogenalkoxy, Alkylthio, Halogenalkylthio, Alkylsulfonyl, Halogenalkylsulfonyl, Alkanylendioxy, Halogenalkanylendioxy oder durch $-O-CHal_2-O-CHal_2-$oder für Alkoxycarbonylamino steht,

$R^4$ für Alkyl, Alkoxyalkyl, Alkylthioalkyl, Cyanalkyl, Fluoralkyl, Alkenyl, Alkinyl, Cycloalkyl oder Alkoxy steht,

$R^5$ für Wasserstoff, Alkyl, Alkoxyalkyl, Alkylthioalkyl, Fluoralkyl, Cyanalkyl, Alkenyl, Alkinyl oder Cycloalkyl steht,

oder

$R^4$ und $R^5$ gemeinsam mit dem Stickstoffatom, an dem sie stehen, für einen gegebenenfalls substituierten Heterocyclus stehen, welcher in der Alkylenkette durch weitere Heteroatome substituiert sein kann,

dadurch gekennzeichnet, daß man

a) ein 2-alkoxycarbonylsubstituiertes 4-Hydroxythiophen-5-carbonamid der Formel (II)

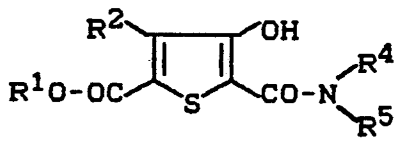

(II)

in welcher

$R^1$, $R^2$, $R^4$ und $R^5$ die obengenannten Bedeutungen haben,

mit einem Acylierungsmittel der Formel (III)

$$X\text{-}R^3 \qquad (III)$$

in der

$R^3$ die vorstehend genannte Bedeutung hat und

X für eine Abgangsgruppe steht,

umsetzt,

oder

b) Verbindungen der Formel (II) mit einem Isocyanat der Formel (IV)

OCN-Y (IV)

in welcher

Y für Alkyl steht, das gegebenenfalls gleich oder verschieden durch Halogen, Cyan, Alkoxycarbonyl, Dialkylaminocarbonyl, Alkanylenaminocarbonyl, Oxaalkanylenaminocarbonyl substituiert ist; für 1-Cyancycloalkyl, Alkoxycarbonyl oder für Phenyl steht, das gegebenenfalls gleich oder verschieden substituiert ist durch Halogen, Alkyl, Halogenalkyl, Alkoxy, Halogenalkoxy, Alkylthio, Halogenalkylthio, Alkylsulfonyl, Halogenalkylsulfonyl, Alkanylendioxy, Halogenalkanylendioxy oder durch den Rest $-O-CHal_2-O-CHal_2-$,

umsetzt,

oder daß man

c) für den Fall, daß $R^3$ ein substituierter Aminocarbonylrest ist, in einer 1. Stufe ein Hydroxythiophencarbonamid der Formel (II) mit Phosgen in Gegenwart einer tertiären organischen Base umsetzt und das gebildete 4-(Chlorcarbonyloxy)-thiophen-5-carbonamid-Derivat der Formel (V)

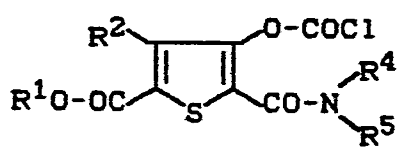

(V)

in der

$R^1$, $R^2$, $R^4$ und $R^5$ die obengenannten Bedeutungen haben,

mit einem Amin der Formel (VI)

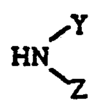

(VI)

in der

Y die oben angegebene Bedeutung hat und

Z für Wasserstoff oder für Alkyl steht oder

Y und Z zusammen für Alkanylen oder Oxyalkanylen stehen,

gegebenenfalls in Gegenwart eines Lösungs- oder Verdünnungsmittels und gegebenenfalls in Gegenwart einer tertiären organischen Base, umsetzt.

6. Schädlingsbekämpfungsmittel, gekennzeichnet durch einen Gehalt an mindestens einem Acyloxythiophen-carbonamid der Formel (I) gemäß den Ansprüchen 1 bis 5.

7. Verfahren zur Bekämpfung von Schädlingen, dadurch gekennzeichnet, daß man Acyloxythiophen-carbonamide der Formel (I) gemäß den Ansprüchen 1 bis 5 auf Schädlinge und/oder ihren Lebensraum einwirken läßt.

8. Verwendung von Acyloxythiophen-carbonamiden der Formel (I) gemäß den Ansprüchen 1 bis 5 zur Bekämpfung von Schädlingen.

9. Verfahren zur Herstellung von Schädlingsbekämpfungsmitteln, dadurch gekennzeichnet, daß man Acyloxythiophen-carbonamide der Formel (I) gemäß den Ansprüchen 1 bis 5 mit Streckmitteln und/oder oberflächenaktiven Mitteln vermischt.